# EUROPEAN PATENT SPECIFICATION

(11) **EP 4 039 282 B1**
(45) Date of publication and mention of the grant of the patent: **04.06.2025**
(21) Application number: 20871241.4
(22) Date of filing: 24.09.2020
(51) Int. Cl.: A61K 48/00, A61P 1/04, A61P 3/10, A61P 9/00, A61P 11/00, A61P 11/06, A61P 11/14, A61P 13/12, A61P 19/02, A61P 25/18, A61P 29/00, A61P 35/00, A61P 37/02, A61P 37/08, A61P 43/00, A61P 17/00, C12N 15/113, A61K 31/7088

(54) **NUCLEIC ACID THAT INHIBITS EXPRESSION OF MEX3B GENE, MEX3B GENE EXPRESSION INHIBITING AGENT, METHOD FOR INHIBITING MEX3B GENE EXPRESSION, AND PROPHYLACTIC OR THERAPEUTIC AGENT FOR DISEASE CAUSED BY MEX3B GENE EXPRESSION**
NUKLEINSÄURE ZUR HEMMUNG DER EXPRESSION DES MEX3B-GENS, MEX3B-GENEXPRESSIONSHEMMER, VERFAHREN ZUR HEMMUNG DER MEX3B-GENEXPRESSION, PROPHYLAKTIKUM ODER THERAPEUTIKUM FÜR ERKRANKUNGEN DURCH MEX3B-GENEXPRESSION
ACIDE NUCLÉIQUE QUI INHIBE L'EXPRESSION DU GÈNE MEX3B, AGENT INHIBANT L'EXPRESSION DU GÈNE MEX3B, PROCÉDÉ D'INHIBITION DE L'EXPRESSION DU GÈNE MEX3B, ET AGENT PROPHYLACTIQUE OU THÉRAPEUTIQUE POUR UNE MALADIE PROVOQUÉE PAR L'EXPRESSION DU GÈNE MEX3B

(30) Priority: 30.09.2019 JP 2019180592
(43) Date of publication of application: 10.08.2022
(73) Proprietor: The University of Tokyo, Bunkyo-ku, Tokyo 113-8654 (JP)
(72) Inventor: AKIYAMA Tetsu, Tokyo 113-8654 (JP); YAMAZUMI Yusuke, Tokyo 113-8654 (JP)
(74) Representative: Appleyard Lees IP LLP
(86) International application number: PCT/JP2020/036105
(87) International publication number: WO 2021/065686

(56) References cited:
- WO-A1-2011/005765
- WO-A1-2017/106382
- WO-A1-2018/008749
- WO-A1-2018/008749
- WO-A1-2018/008750
- WO-A1-2018/008750
- JP-A- 2017 212 911
- JP-A- 2018 011 593
- YAMAZUMI YUSUKE ET AL: "The RNA-binding protein Mex-3B plays critical roles in the development of steroid-resistant neutrophilic airway inflammation", BIOCHEMICAL AND BIOPHYSICAL RESEARCH COMMUNICATIONS, ELSEVIER, AMSTERDAM NL, vol. 519, no. 2, 4 September 2019 (2019-09-04), pages 220 - 226, XP085840267, ISSN: 0006-291X, [retrieved on 20190904], DOI: 10.1016/J.BBRC.2019.08.158
- YAMAZUMI, YUSUKE ET AL.: "The RNA-binding protein Mex-3B plays critical roles in the development of steroid -resistant neutrophilic airway inflammation", BIOCHEMICAL AND BIOPHYSICAL RESEARCH COMMUNICATIONS, vol. 519, 4 September 2019 (2019-09-04), pages 220 - 226, XP085840267, DOI: 10.1016/j.bbrc.2019.08.158
- YAMAZUMI, YUSUKE ET AL.: "The RNA Binding Protein Mex-3B Is Required for IL -33 Induction in the Development of Allergic Airway Inflammation", CELL REPORTS, vol. 16, 2016, pages 2456 - 2471, XP055450972, DOI: 10.1016/j.celrep.2016.07.062

## Description

### TECHNICAL FIELD

The present invention relates to a nucleic acid that inhibits MEX3B gene expression; an MEX3B gene expression inhibitor including such a nucleic acid; a method for inhibiting MEX3B gene expression; and a prophylactic or therapeutic agent for a disease caused by MEX3B gene expression.

### BACKGROUND ART

In recent years, an increase in expression of inflammatory cytokines or chemokines, such as IL-6, IL-13, TNF, G-CSF, CXCL1, CXCL2, or CXCL5, has been revealed to be involved in serious diseases (e.g., severe asthma, joint diseases, diabetes, inflammatory bowel diseases, atopic dermatitis, systemic erythematosus, cancer, psychiatric diseases, cardiovascular diseases, respiratory diseases, type 2 diabetes, renal diseases) (see, for example, Non-Patent Document 1). The MEX3B protein is known to be a molecule that binds to a variety of mRNAs to control their function (specifically, for translation to proteins) or stability (see, for example, Non-Patent Document 2). Patent Document 1 discloses that an MEX3B protein binds to mRNAs for inflammatory cytokines or chemokines to affect their function (specifically, for translation to a protein) or stability and thus to be involved in the development of diseases caused by inflammatory cytokines or chemokines. Thus, the inhibition of MEX3B expression is potentially important for treating or preventing such diseases.

Patent Document 1: PCT International Publication No. WO2018/08750A1 relates to a pharmaceutical composition for treating a disease or condition selected from the group consisting of interleukin 6 (IL-6), interleukin 13 (IL-13), TNF (tumor necrosis factor), G-CSF (granulocyte- 6, IL-13, TNF, G-CSF, CXCL 1, CXCL 2, or CXCL 1, CXCL 1, CXCL 2, or CXCL 5, or screening agents for preventing or treating diseases caused by CXCL 1, CXCL 2, to a preventive or therapeutic agent for diseases caused by CXCL5. It does not disclose or suggest a nucleic acid according to the appended claims.

Patent Document 2: PCT International Publication No. WO2018/08749A1 relates to a nucleic acid that suppresses the expression of the MEX3B gene, a MEX3B gene expression inhibitor comprising the nucleic acid, a method of inhibiting MEX3B gene expression, and a preventive or therapeutic agent for disease caused by MEX3B gene expression. It does not disclose or suggest a nucleic acid according to the appended claims.

Patent Document 3: PCT International Publication No. WO2017/106382A1 relates to methods and compositions for increasing the expression of gene products for which increased expression can provide benefit in central nervous system diseases or conditions. It does not disclose or suggest a nucleic acid according to the appended claims.

Patent Document 4: PCT International Publication No. WO2011/005765A1 relates to methods for producing a biological product in an embryonated egg by introducing into the egg a RNA effector molecule capable of modulating expression of a target gene, wherein the modulation enhances production of the biological product in the egg. It does not disclose or suggest a nucleic acid according to the appended claims.

Non-Patent Document 1: Int Immunol. 2015 Jan; 27(1):21-9 relates to the therapeutic uses of anti-interleukin-6 receptor antibody. It does not disclose or suggest a nucleic acid according to the appended claims.

Non-Patent Document 2 : Cancer Discov. 2016 Jan;6(1):80-95 discloses YAP-driven MDSC recruitment via heterotypic CXCL5-CXCR2 signaling reveals an effective therapeutic strategy for advanced prostate cancer. It does not disclose or suggest a nucleic acid according to the appended claims.

Non-Patent Document 3: Nucleic Acids Res. 2007;35(4):1289-300 discloses hMex-3 proteins constitute a novel family of evolutionarily conserved RNA-binding proteins, differentially recruited to P bodies and potentially involved in post-transcriptional regulatory mechanisms. It does not disclose or suggest a nucleic acid according to the appended claims. Non-Patent Document 4 : Biochem Biophys Res Commun. 2019 Nov 5;519(2):220-226 discloses knockdown of MEX3B by LNA antisense oligonucleotide suppresses neutrophilic allergic airway inflammation. The citation provides alternative MEX3B targetting oligonucleotides for inhibiting expression of MEXB gene. It does not disclose or suggest a nucleic acid according to the appended claims.

### DISCLOSURE OF THE INVENTION

### Problems to be Solved by the Invention

Patent Document 2 discloses nucleic acids having low cytotoxicity and being capable of inhibiting MEX3B gene expression. Unfortunately, such nucleic acids have room for improvement in the MEX3B gene expression-inhibiting effect.

The present invention has been made in light of the circumstances mentioned above, and an object of the present invention is to provide a nucleic acid having low cytotoxicity and an improved ability to inhibit MEX3B gene expression; an MEX3B gene expression inhibitor comprising such a nucleic acid; a method for inhibiting MEX3B gene expression; and a prophylactic or therapeutic agent for diseases caused by MEX3B gene expression.

### Means for Solving the Problems

The inventors have designed a large number of antisense oligonucleotides, which cover the full range of unspliced human MEX3B pre-mRNA, and comprehensively subjected them to testing for MEX3B gene expression inhibition. As a result, the inventors have found that specific antisense oligonucleotides having sequences complementary to specific sequences in the MEX3B gene are significantly effective in inhibiting the expression of mRNA of the MEX3B gene and are also significantly effective in inhibiting MEX3B protein expression. The present invention has been completed based on the findings. Specifically, the present invention is as set out in the appended claims.

The first aspect of the present invention is directed to a nucleic acid selected from the following oligonucleotides:
(1) an oligonucleotide consisting of the nucleotide (base) sequence set forth in SEQ ID NO: 1 or 2 in the Sequence Listing;
(2) an antisense oligonucleotide that consists of a nucleotide sequence comprising a deletion, substitution, and/or addition of one or two nucleotides with respect to the nucleotide sequence set forth in SEQ ID NO: 1 or 2 in the Sequence Listing and inhibits MEX3B gene expression; and
(3) an antisense oligonucleotide that comprise the sequence set forth in SEQ ID NO: 3 in the Sequence Listing and inhibits MEX3B gene expression.

The second aspect of the present invention is directed to an MEX3B gene expression inhibitor comprising the nucleic acid according to the first aspect.

The third aspect of the present disclosure is directed to a method for inhibiting MEX3B gene expression, the method comprising bringing the inhibitor according to the second aspect into contact with a subject, excluding a human individual.

The fourth aspect of the present invention is directed to a prophylactic or therapeutic agent for a disease caused by MEX3B gene expression, the agent comprising the inhibitor according to the second aspect.

### Effects of the Invention

The present invention makes it possible to provide a nucleic acid having low cytotoxicity and an improved ability to inhibit MEX3B gene expression; an MEX3B gene expression inhibitor comprising such a nucleic acid; a method for inhibiting MEX3B gene expression; and a prophylactic or therapeutic agent for a disease caused by MEX3B gene expression.

### BRIEF DESCRIPTION OF THE DRAWINGS

FIG. 1 is a graph showing the reduction of the human MEX3B mRNA expression level by transfection with each of gapmer-type nucleic acids;
FIG. 2 is a graph showing the reduction of the human MEX3B mRNA expression level by transfection with each of gapmer-type nucleic acids;
FIG. 3 is a graph showing the reduction of the human MEX3B mRNA expression level by transfection with each of gapmer-type nucleic acids;
FIG. 4 is a graph showing the reduction of the mouse MEX3B mRNA expression level by transfection with each of gapmer-type nucleic acids;
FIG. 5 is a graph showing a result of the inhibition of the human MEX3B mRNA expression through free uptake of each of gapmer-type nucleic acids;
FIG. 6 is a view showing a result of the inhibition of human and mouse MEX3B protein expression by transfection with each of gapmer-type nucleic acids;
FIG. 7 is a view showing a result of the inhibition of human MEX3B protein expression by transfection with each of gapmer-type nucleic acids;
FIG. 8 is a view showing a result of the inhibition of mouse MEX3B protein expression by transfection with each of gapmer-type nucleic acids;
FIG. 9 is a view showing a result of the inhibition of human MEX3B protein expression by transfection with each of gapmer-type nucleic acids; and
FIG. 10 is a view showing a result of the inhibition of mouse MEX3B protein expression by transfection with each of gapmer-type nucleic acids.

### PREFERRED MODE FOR CARRYING OUT THE INVENTION

### MEX3B Gene

The MEX3B gene includes exon 1, an intron, and exon 2, and such a structure is highly conserved among humans, mice, and other mammals. The untranslated regions (UTRs) not encoding amino acids in the exons include 5'UTR upstream of the initiation codon and 3'UTR downstream of the termination codon. The human MEX3B gene encoding the human MEX3B mRNA has the sequence set forth in SEQ ID NO: 4 shown later. In SEQ ID NO: 4, the sequence from nucleotide 437 to 2146 is CDS, the sequence from nucleotide 1 to 436 is 5'UTR, and the sequence from nucleotide 2147 to 3532 is 3'UTR. The mouse MEX3B gene encoding the mouse MEX3B mRNA has the sequence set forth in SEQ ID NO: 5 shown later. In SEQ ID NO: 5, the sequence from nucleotide 319 to 2049 is CDS, the sequence from nucleotide 1 to 318 is 5'UTR, and the sequence from nucleotide 2050 to 3416 is 3'UTR. SEQ ID NO: 6 shown later is the 836-nucleotide sequence of the intronic region in the human MEX3B gene. SEQ ID NO: 7 is the sequence encoding unspliced human MEX3B pre-mRNA. In the sequence of SEQ ID NO: 7 encoding human MEX3B pre-mRNA, the sequence from nucleotide 437 to 692 and the sequence from nucleotide 1529 to 2982 are each CDS, the sequence from nucleotide 1 to 436 is 5'UTR, the sequence from nucleotide 2983 to 4368 is 3'UTR, and the region from nucleotide 693 to 1528 corresponds to the intronic region represented by SEQ ID NO: 6 in the human MEX3B gene. Genes encoding MEX3B proteins (e.g., a protein having an amino acid sequence represented by SEQ ID NO: 8 (human) or 9 (mouse)) all belong to the MEX3B gene.

### Acquisition of MEX3B Gene

Any method may be used to acquire the MEX3B gene. Suitable probes or primers may be prepared based on information indicating the nucleotide sequences of SEQ ID NOS: 4, 5, 6, and 7 and the amino acid sequences of SEQ ID NOS: 8 and 9 in the Sequence Listing provided herein, and then used to select desired clones from a human cDNA library, which is prepared from suitable MEX3B gene-expressing cells by a conventional method, when the MEX3B gene is isolated.

### Nucleic Acid That Inhibits MEX3B Gene Expression

The nucleic acid that exhibits MEX3B gene expression according to the first aspect (hereinafter also simply referred to as "the nucleic acid according to the first aspect) is any one of the oligonucleotides (1), (2), and (3) shown below.
(1) An oligonucleotide consisting of the sequence set forth in SEQ ID NO: 1 or 2 in the Sequence Listing.
(2) An antisense oligonucleotide that consists of the sequence having a deletion, substitution, and/or addition of one or two nucleotides with respect to the sequence set forth in SEQ ID NO: 1 or 2 in the Sequence Listing and inhibits MEX3B gene expression.
(3) An antisense oligonucleotide that comprises the sequence set forth in SEQ ID NO: 3 in the Sequence Listing and inhibits MEX3B gene expression.

The nucleic acid according to the first aspect may inhibit MEX3B gene expression by reducing the level of either or both of mRNA and protein. The inhibition may be at any level as long as at least mRNA level and/or the protein level is reduced as compared to the negative control level (or wild type level). Specifically, at least mRNA level and/or the protein level is preferably reduced by 10% or more, more preferably by 15% or more, even more preferably by 20% or more, further more preferably by 30% or more, still more preferably by 40% or more, yet more preferably by 50% or more, and most preferably by 60% or more as compared to the negative control level (or wild type level). Regarding the oligonucleotide (1), the sequence (GCGGATACTACAGCTT) set forth in SEQ ID NO: 1 and the sequence (GAGCGGATACTACAGC) set forth in SEQ ID NO: 2 are the sequences of the gapmer-type oligonucleotides: hmrGD-89-2 and hmrGD-89, respectively, which are shown later in the Examples section.

Regarding the oligonucleotide (2), the sequence has a deletion, substitution, and/or addition of one or two nucleotides and preferably has a deletion, substitution, and/or addition of one nucleotide with respect to the sequence set forth in SEQ ID NO: 1 or 2. The antisense oligonucleotide (2) preferably has a length of 14 nucleotides or more and more preferably has a length of 15 nucleotides or more. The length of the antisense oligonucleotide (2) preferably has an upper limit of 18 nucleotides or less, more preferably 17 nucleotides or less, even more preferably 16 nucleotides or less. The nucleotide sequence has a deletion, substitution, and/or addition of one or two nucleotides. In view of producibility, the nucleotide sequence preferably has a deletion and/or substitution of one or two nucleotides, and more preferably has a deletion of one or two nucleotides. Regarding the DNA mutation level, for example, the variant oligonucleotide preferably has a sequence identity of 90% or more, more preferably 93% or more, even more preferably 95% or more, furthermore preferably 97% or more, most preferably 98% or more with respect to the sequence set forth in SEQ ID NO: 1 or 2 in the Sequence Listing. The DNA sequence set forth in SEQ ID NO: 1 or 2 in the Sequence Listing may be partially modified by any of various artificial processes including, for example, site-directed mutagenesis, random mutation by treatment with a mutagenic agent, and mutation, deletion, or ligation of DNA fragments through restriction enzyme cleavage.

Regarding the oligonucleotide (3), the sequence (GCGGATACTACAGC) set forth in SEQ ID NO: 3 is a 14- nucleotide sequence common to SEQ ID NOS: 1 and 2 shown above. Regarding the oligonucleotide (3), the antisense oligonucleotide preferably has a sequence complementary to an oligonucleotide comprising at least 12 contiguous nucleotides in the sequence of the MEX3B gene, more preferably has a sequence complementary to an oligonucleotide comprising at least 13 contiguous nucleotides in the sequence of the MEX3B gene, and even more preferably has a sequence complementary to an oligonucleotide comprising at least 14 contiguous nucleotides in the nucleotide sequence of the MEX3B gene. Regarding the upper limit of the length of the antisense oligonucleotide, the antisense oligonucleotide preferably has a sequence complementary to an oligonucleotide comprising at most 25 contiguous nucleotides in the sequence of the MEX3B gene, more preferably has a sequence complementary to an oligonucleotide comprising at most 20 contiguous nucleotides in the nucleotide sequence of the MEX3B gene, even more preferably has a sequence complementary to an oligonucleotide comprising at most 19 contiguous nucleotides in the sequence of the MEX3B gene, further more preferably has a sequence complementary to an oligonucleotide comprising at most 18 contiguous nucleotides in the sequence of the MEX3B gene, still more preferably has a sequence complementary to an oligonucleotide comprising at most 17 contiguous nucleotides in the sequence of the MEX3B gene, and yet more preferably has a sequence complementary to an oligonucleotide comprising at most 16 contiguous nucleotides in the nucleotide sequence of the MEX3B gene.

In view of producibility and other properties, the antisense oligonucleotide (3) preferably has a sequence of 15 nucleotides or less. Regarding the level of DNA mutation of the oligonucleotide (3), for example, the variant oligonucleotide preferably has a sequence identity of 90% or more, more preferably 93% or more, even more preferably 95% or more, furthermore preferably 97% or more, most preferably 98% or more with respect to the sequence set forth in SEQ ID NO: 3 in the Sequence Listing.

The antisense oligonucleotide (1), (2), or (3) is preferably, for example, such that after being introduced into cells, it can hybridize to a region in the MEX3B gene, which is complementary to it, and that the nucleotide chain-containing MEX3B mRNA can be degraded by a nuclease (e.g., RNase H) specific for a hybrid duplex resulting from the hybridization. The antisense oligonucleotide may be DNA or RNA. Preferably, the antisense oligonucleotide is DNA for the mRNA cleavage by the specific nuclease.

The antisense oligonucleotide (1), (2), or (3) preferably contains at least one nucleotide having at least one structure selected from the group consisting of a phosphorothioate structure, a crosslinked structure, and an alkoxy structure. For example, the antisense oligonucleotide in which a phosphodiester bond moiety linking nucleotides has a phosphorothioate structure can have nuclease resistance and improved hydrophobicity, which will improve its uptake into the cells or their nuclei. When the nucleotide sugar moiety has a crosslinked structure, such as 2',4'-BNA (2',4'-bridged nucleic acid also called LNA (locked nucleic acid)) or ENA (2'-O,4'-C-ethylene-bridged nucleic acid), or an alkoxy structure, such as a 2'-O-methyl or 2'-O-methoxyethyl (2'-MOE) moiety, the antisense oligonucleotide can have nuclease resistance and an improved ability to bind to mRNA.

In the antisense oligonucleotide, at least one phosphodiester bond moiety linking nucleotides preferably has a phosphorothioate structure. In the antisense oligonucleotide, preferably at least 50%, more preferably at least 70%, even more preferably at least 90%, most preferably all of the phosphodiester bonds have a phosphorothioate structure. In the antisense oligonucleotide, at least one of the terminal nucleotides preferably has a crosslinked structure or an alkoxy structure. More specifically, preferably at least one of nucleotides from the terminal end to the fourth nucleotide on one terminal side, more preferably at least one of nucleotides from the terminal end to the third nucleotide on one terminal side, even more preferably at least one of nucleotides from the terminal end to the second or third nucleotide on one terminal side has a crosslinked structure or an alkoxy structure. Each terminal end of the antisense oligonucleotide preferably has a crosslinked structure or an alkoxy structure (what is called gapmer-type antisense oligonucleotides). Preferably at least one of nucleotides from the terminal end to the fourth nucleotide on each of both terminal sides respectively, more preferably at least one of nucleotides from the terminal end to the third nucleotide on each of both terminal sides respectively, even more preferably at least one of nucleotides from the terminal end to the second or third nucleotide on each of both terminal sides respectively has a crosslinked structure or an alkoxy structure. The nucleic acid according to the first aspect may be produced by a common method using a DNA synthesizer and a known organic synthesis technique.

The nucleic acid according to the first aspect of the disclosure may be introduced into cells in vitro or in vivo by the contact with cells, for example, by adding it to the medium for any desired cells. When having at least one selected from the group consisting of a phosphorothioate structure, a crosslinked structure, and an alkoxy structure, the nucleic acid according to the first aspect can be more effectively introduced into cells. When having at least one selected from the group consisting of a phosphorothioate structure, a crosslinked structure, and an alkoxy structure, the nucleic acid according to the first aspect may be used in combination with the lipofection vehicle described later. In this case, the introduction of the nucleic acid into cells can be further improved. As shown later, however, the nucleic acid according to the first aspect has such a strong MEX3B gene expression-inhibiting effect that there will be no need to use the lipofection vehicle. Moreover, as shown in Example 4 below, free uptake of the nucleic acid according to the first aspect into cells is possible.

A method for introducing the nucleic acid according to the first aspect into cells may include inserting the nucleic acid into a suitable vector; and then introducing the vector into suitable host cells. The suitable vector may be any type, such as autonomously replicating vectors (e.g., plasmids). Preferably, after being introduced into host cells, the vector can be incorporated into the genome of the host cell and replicated together with the host chromosome. Examples of available suitable vectors include plasmids derived from E. coli (e.g., pBR322, pUC118), plasmids derived from B. subtilis (e.g., pUB110, pSH19), and animal viruses, such as bacteriophages, retroviruses, and vaccinia viruses. In the recombination, a translation initiation codon and a translation termination codon may be added using suitable synthetic DNA adapters.

If necessary, the nucleic acid according to the first aspect may be operably linked to a suitable terminator, such as a human growth hormone terminator or a TPI1 or ADH3 terminator for fungal hosts. The recombinant vector may contain additional elements, such as a polyadenylation signal (e.g., one derived from SV40 or adenoviral 5E1b region), a transcription enhancer sequence (e.g., SV40 enhancer), and a translational enhancer sequence (e.g., one encoding an adenovirus VA RNA). The recombinant vector may further include a DNA sequence that allows the vector to replicate in the host cell, an example of which is an SV40 replication origin (for mammalian host cells). The recombinant vector may further contain a selection marker. The selection marker may be a gene for which the complement is missing in the host cell, such as a dihydrofolate reductase (DHFR) gene or a Schizosaccharomyces pombe TPI gene, or a drug resistance gene, such as a gene resistant to ampicillin, kanamycin, tetracycline, chloramphenicol, neomycin, or hygromycin.

The host cells into which the nucleic acid according to the first aspect or a vector containing the nucleic acid is to be introduced may be higher eukaryotic cells, bacterial cells, yeast cells, fungal cells, or other cells, and are preferably mammalian cells. Examples of mammalian cells include HEK293 cells, HeLa cells, COS cells (e.g., COS-7 cells), BHK cells, CHL or CHO cells, and BALB/c mouse cells (e.g., BALB/c mouse fetal fibroblasts). Known methods, such as lipofection, electroporation, and calcium phosphate method, may be used to introduce the gene into mammalian cells and to transform the cells for the expression of the gene.

The MEX3B gene expression inhibition by the nucleic acid according to the first aspect of the disclosure may be detected in vivo or in vitro using, for example, a probe or primer having part or the whole of the nucleotide sequence of the MEX3B gene, which is based on the information on the nucleotide sequence of the MEX3B gene. In particular, the level of mRNA expression of the MEX3B gene may be measured by RT-PCR, Northern blotting, or other conventional methods.

When PCR is performed, any primers capable of specifically amplifying the MEX3B gene may be used, which may be appropriately selected based on the MEX3B gene sequence information. Examples of available probes or primers include oligonucleotides comprising at least 10 contiguous nucleotides derived from the MEX3B gene; and antisense oligonucleotides having sequences complementary to such oligonucleotides. More specifically, oligonucleotides having a sequence of 10 to 60 contiguous nucleotides, preferably 10 to 40 contiguous nucleotides derived from the MEX3B gene may be used, and antisense oligonucleotides having sequences complementary to such oligonucleotides may be used.

The level of MEX3B protein expression may be measured by common immunoassay, such as Western blotting or ELISA. Specifically, the protein expression level may be measured by common methods known to those skilled in the art, such as those described in Molecular Cloning 2nd Edition or Current Protocols in Molecular Biology.

### MEX3B Gene Expression Inhibitor

The MEX3B gene expression inhibitor according to the second aspect (hereinafter, also simply referred to as "the inhibitor according to the second aspect") includes the nucleic acid according to the first aspect. As mentioned above, the MEX3B expression inhibition may correspond to at least the reduction in transcription level and/or the reduction in protein expression level. To be more effectively introduced into cells, the MEX3B gene expression inhibitor according to the second aspect may further include a lipofection vehicle. Alternatively, the inhibitor may be free of a lipofection vehicle because free uptake of the nucleic acid according to the first aspect is possible as shown in Example 4 below and a strong MEX3B gene expression-inhibiting effect can be achieved in that case. The lipofection vehicle may be a vehicle having a high affinity for cell membranes (e.g., liposomes, cholesterols), and is preferably Lipofectamine or Lipofectin, more preferably Lipofectamine. When having at least one selected from the group consisting of a phosphorothioate structure, a crosslinked structure, and an alkoxy structure, the nucleic acid according to the first aspect may be used in combination with the lipofection vehicle, so that it can be more effectively introduced into cells. For example, the nucleic acid according to the first aspect having at least one selected from the group consisting of a phosphorothioate structure, a crosslinked structure, and an alkoxy structure may be brought into contact with a subject, which will be described later, in the presence of the lipofection vehicle.

The inhibitor according to the second aspect may be in the form of a mixture including the nucleic acid according to the first aspect, the lipofection vehicle, and other optional components (e.g., water, buffer solution). Alternatively, the inhibitor may be in the form of a kit including the nucleic acid according to the first aspectand other optional components, which are contained in a separate vessel, and the lipofection vehicle and other optional components, which are contained in another separate vessel. The inhibitor according to the second aspect may be in any dosage form, such as a liquid, a granule, a tablet, a capsule, or a patch. In the in vivo case, biological tissues may be directly exposed to the inhibitor according to the second aspect. More preferably, the inhibitor may be applied (e.g., in the form of a liquid) or administered in vivo orally, intravascularly (intravenously or intraarterially), intraorally, sublingually, intrarectally, intraperitoneally, intravaginally, intraspinally, intramuscularly, dermally, subdermally, intradermally, intravesically, intratracheally (intrabronchially), ocularly, nasally, or intra-aurally by injection, spray, application, or any other means.

### Method for Inhibiting MEX3B Gene Expression

The method for inhibiting MEX3B gene expression according to the third aspect of the disclosure includes bringing the inhibitor according to the second aspect into contact with a subject. As shown in Example 4 below, the inhibitor according to the second aspect can be introduced into cells through free uptake, for example, without the need for calcium shock or the like, when brough into contact with the cells. Examples of the subject include individual organisms (e.g., individual mammals or birds), microorganisms, protozoans, biological tissues, biological tissue sections, human cells, and animal cells. Examples of mammals and birds include cattle, sheep, goats, pigs, horses, dogs, chickens, mice, rats, guinea pigs, hamsters, rabbits, and primates. Individual primates may be monkeys, and may or may not be humans. The inhibitor according to the second aspect may be brought into contact with the subject in any manner. For example, the inhibitor according to the second aspect may be added to a medium containing the subject, or a medium containing the inhibitor according to the second aspect may be prepared in advance and then brought into contact with the subject. The contact may be performed at any temperature for any time period under any conditions. The contact temperature, the contact time period, and other contact conditions may be appropriately selected depending on the type of the subject and other factors.

### Prophylactic or Therapeutic Agent for Diseases Caused by MEX3B Gene Expression

The prophylactic or therapeutic agent according to the fourth aspect for a disease caused by MEX3B gene expression includes the MEX3B gene expression inhibitor according to the second aspect. Interleukin 6 (IL-6) is a key cytokine involved in inflammation, hematopoiesis, bone metabolism, exacerbation of tumor, and so on. The activity of IL-6 is known to contribute generally to the transition from acute inflammation to acquired immune response, the development of chronic inflammatory diseases, and so on (see, for example, J Asthma. 2008;45 Suppl 1:41-4). Interleukin 13 (IL-13) is known to play a role as an inflammatory cytokine in enhancing allergic inflammation in peripheral tissues. IL-13 is known to be involved not only in the enhancement of allergic response, which is a main cause of allergic asthma, but also in refractoriness to asthma therapies, in other words, resistance to steroids. IL-13 is also involved in the pathogenesis not only of asthma but also of inflammatory bowel diseases and atopic dermatitis (e.g., J Allergy (Cairo). 2012;2012:316049, N Engl J Med 2011;365:1088-1098). Among the tumor necrosis factors (TNFs), TNF-α is an important factor for protection against infection because it is a signaling factor that triggers inflammatory responses. TNFs are also known to be involved in disorders caused by increased inflammation at the same time. That is, TNFs are involved in exacerbation of the state of various diseases mainly including joint diseases (e.g., rheumatoid arthritis, psoriatic arthritis, spondylarthrosis, ankylosing spondylitis), inflammatory bowel diseases (e.g., ulcerative colitis, Crohn's disease), cancer (e.g., ovarian cancer, breast cancer), psychiatric disorders (e.g., depression, bipolar disorder, epilepsy, Alzheimer's disease, Parkinson's disease, multiple sclerosis), cardiovascular diseases (e.g., heart failure, arteriosclerosis), respiratory diseases (e.g., bronchial asthma, chronic bronchitis, chronic obstructive pulmonary disease, acute lung injury), type 2 diabetes, and renal diseases (e.g., ischemic kidney injury, post-transplantation rejection, glomerulonephritis) (see, for example, J Allergy Clin Immunol. 2008 Jan;121(1):5-10, J Pathol. 2008 Jan;214(2):149-60). Granulocyte-colony stimulating factor (G-CSF) is known to act to promote granulocyte production and to enhance neutrophil function. IL-13, TNF, and G-CSF are also known to be involved in the severity of asthma (see, for example, Curr Opin Immunol. 2013 Dec;25(6):755-60).

CXCL1, CXCL2, and CXCL5 belong to the inflammatory chemokine CXC subfamily. The secretion of CXCL1, CXCL2, and CXCL5 in lung tissues due to excessively increased inflammation in the airway mucosa promotes the invasion of neutrophils, which express a high level of CXCR2, a receptor for CXCL1, CXCL2, and CXCL5. Consequently, the invasion of steroid-resistant neutrophils triggers chronic inflammation, which induces irreversible airway remodeling resulting from the occurrence of severe asthma. The inventors have found that the MEX3B gene is involved in the development of diseases caused by IL-6, IL-13, TNF, G-CSF, CXCL1, CXCL2, or CXCL5. Thus, the prophylactic or therapeutic agent according to the fourth aspect would be effective in preventing or treating diseases caused by increased expression (e.g., excessively increased expression, preferably higher expression than normal expression, more preferably excessively higher expression than normal expression) of IL-6, IL-13, TNF, G-CSF, CXCL1, CXCL2, or CXCL5, such as severe asthma, chronic obstructive pulmonary disease, rheumatoid arthritis, colitis, Crohn's disease, atopic dermatitis, systemic erythematosus, and cancer, in particular, severe asthma, chronic obstructive pulmonary disease, rheumatoid arthritis, colitis, Crohn's disease, atopic dermatitis, systemic erythematosus, and cancer attributable to IL-6, IL-13, TNF, G-CSF, CXCL1, CXCL2, or CXCL5 (Int Immunol. 2015 Jan;27(1):21-9, Cancer Discov. 2016 Jan;6(1): 80-95).

It is known that the MEX3B protein induces apoptosis (see, for example, Japanese Patent No. 4429269). Apoptosis is known to be involved not only in normal physiological processes but also in the development of serious diseases, such as neurodegenerative diseases. For example, neurodegenerative diseases (e.g., Alzheimer's disease, Parkinson's disease, amyotrophic lateral sclerosis, Huntington's chorea) are thought to be caused by abnormally increased apoptosis (Wolozin, B., et al. (1996) Science, 274, 1710-1713). Thus, the prophylactic or therapeutic agent according to the fourth aspect would be effective in preventing or treating neurodegenerative diseases.

The prophylactic or therapeutic agent according to the fourth aspect may be administered orally, parenterally, systemically, or topically. Parenteral administration methods include intravenous injection, such as infusion, intramuscular injection, intraperitoneal injection, and subcutaneous injection. An appropriate administration method may be selected depending on the age and symptoms of the patient. The dosage may vary depending on the age, the administration route, and the number of times of administration. Those skilled in the art can select an appropriate dosage depending on them. Formulations suitable for parenteral administration include those containing an additive, such as a stabilizer, a buffer, a preservative, or an isotonic agent, which may further contain a pharmaceutically acceptable carrier or additive. Examples of such a carrier and additive include, but are not limited to, water, organic solvents, polymeric compounds (e.g., collagen, polyvinyl alcohol), stearic acid, human serum albumin (HSA), mannitol, sorbitol, lactose, and surfactants.

The dosage of the nucleic acid according to the first aspect as an active ingredient may be usually in the range of about 0.1 µg to about 100 mg per kg body weight per dose.

### EXAMPLES

Hereinafter, the present invention will be described more specifically with reference to examples.

### Example 1

### Preparation of Gapmer-Type Nucleic Acids

A large number of antisense oligonucleotides shifted by 5 nucleotides from one another were prepared to cover the full range of unspliced human MEX3B pre-mRNA, and then comprehensively subjected to testing for MEX3B gene expression inhibition. Among the large number of gapmer-type nucleic acids prepared as the antisense oligonucleotides shifted by 5 nucleotides from one another and complementary to the human MEX3B pre-mRNA, Table 1 below shows gapmer-type nucleic acids hmrGD-87 (SEQ ID NO: 11), hmrGD-88 (SEQ ID NO: 12), hmrGD-89 (SEQ ID NO: 2), hmrGD-90 (SEQ ID NO: 13), hmrGD-91 (SEQ ID NO: 14), hmrGD-92 (SEQ ID NO: 15), hmrGD-93 (SEQ ID NO: 16), hmrGD-176 (SEQ ID NO: 17), hmrGD-178 (SEQ ID NO: 18), hmrGD-179 (SEQ ID NO: 19), hmrGD-180 (SEQ ID NO: 20), hmrGD-182 (SEQ ID NO: 21), hmrGD-199 (SEQ ID NO: 22), hmrGD-201 (SEQ ID NO: 23), hmrGD-202 (SEQ ID NO: 24), hmrGD-203 (SEQ ID NO: 25), and hmrGD-205 (SEQ ID NO: 26). Among those gapmer-type nucleic acids, hmrGD-176, hmrGD-178, hmrGD-179, hmrGD-180, hmrGD-182, hmrGD-199, hmrGD-201, hmrGD-202, hmrGD-203, and hmrGD-205 are disclosed in Patent Document 2. The negative control used was gapmer-type nucleic acid LNA-NC(2,2) (SEQ ID NO: 10). The positive control used was gapmer-type nucleic acid LNA-1(2,2), which was an antisense oligonucleotide complementary to a given target sequence on the human pre-mRNA and had been confirmed to show a certain level of MEX3B gene expression-inhibiting effect. A 2- or 3-base LNA (2',4'-BNA) was attached to each end of each of the gapmer-type nucleic acids. The other nucleotides between them were normal DNA, and the phosphodiester bond between nucleotides was phosphorothioated. The gapmer-type nucleic acid as the negative control had a total length of 15 nucleotides, and the other gapmer-type nucleic acids each had a total length of 16 nucleotides. The expression "(2,2)" means that the two nucleotides at each end of the gapmer-type nucleic acid are LNA.

**[Table 1]**

| Gapmer-type nucleic acid | SEQ ID NO | Gapmer sequence | Oligonucleo tide length | | Target sequence | Target region (Human pre-mRNA) | Target region |
|---|---|---|---|---|---|---|---|
| Negative control | 10 | AACACGTCTATACGC | 15 | | - | - | - |
| hmrGD-87 | 11 | TACAGCTTTCCAAGCT | 16 | | AGCTTGGAAAGCTGTA | 3129-3144 | 3'UTR |
| hmrGD-88 | 12 | GATACTACAGCTTTCC | 16 | | GGAAAGCTGTAGTATC | 3134-3149 | 3'UTR |
| hmrGD-89 | 2 | GAGCGGATACTACAGC | 16 | | GCTGTAGTATCCGCTC | 3139-3154 | 3'UTR |
| hmrGD-90 | 13 | AAAATGAGCGGATACT | 16 | | AGTATCCGCTCATTTT | 3144-3159 | 3'UTR |
| hmrGD-91 | 14 | ATTTATAGAGCATGCA | 16 | | TGCATGCTCTATAAAT | 3243-3258 | 3'UTR |
| hmrGD-92 | 15 | CTATTATTTATAGAGC | 16 | | GCTCTATAAATAATAG | 3248-3263 | 3'UTR |
| hmrGD-93 | 16 | CGTTCCTATTATTTAT | 16 | | ATAAATAATAGGAACG | 3253-3268 | 3'UTR |
| hmrGD-176 | 17 | GTACATTTAAGCTCAA | 16 | | TTGAGCTTAAATGTAC | 4119-4134 | 3'UTR |
| hmrGD-178 | 18 | GCTCAGTAAAGTACAT | 16 | | ATGTACTTTACTGAGC | 4129-4144 | 3'UTR |
| hmrGD-179 | 19 | ACTTTGCTCAGTAAAG | 16 | | CTTTACTGAGCAAAGT | 4134-4149 | 3'UTR |
| hmrGD-180 | 20 | TTTAAACTTTGCTCAG | 16 | | CTGAGCAAAGTTTAAA | 4139-4154 | 3'UTR |
| hmrGD-182 | 21 | CATAAAATAAAATATA | 16 | | TATATTTTATTTTATG | 4163-4178 | 3'UTR |
| hmrGD-199 | 22 | AAAAATAAAACTCTTG | 16 | | CAAGAGTTTTATTTTT | 4248-4263 | 3'UTR |
| hmrGD-201 | 23 | TAAATGTCAGAAAAAT | 16 | | ATTTTTCTGACATTTA | 4258-4273 | 3'UTR |
| hmrGD-202 | 24 | AACTTTAAATGTCAGA | 16 | | TCTGACATTTAAAGTT | 4263-4278 | 3'UTR |
| hmrGD-203 | 25 | TGTAGAACTTTAAATG | 16 | | CATTTAAAGTTCTACA | 4268-4283 | 3'UTR |
| hmrGD-205 | 26 | ACCTTTATTATGTAGA | 16 | | TCTACATAATAAAGGT | 4278-4293 | 3'UTR |

### Transfection with Gapmer-Type Nucleic Acid

Each of the gapmer-type nucleic acids of Examples and Comparative Examples was used to transfect (introduced into) cells. A549 human alveolar basal epithelial adenocarcinoma cells were transfected with each gapmer-type nucleic acid. The gapmer-type nucleic acid was introduced at a final concentration of 20 nM into the cells using Lipofectamine RNAiMAX (manufactured by Invitrogen) and its recommended protocol.

### Quantitative RT-PCR Test

Cells were observed for their condition under a microscope 48 hours after the transfection and then harvested. The total RNA was then collected from the cells using a lysis buffer TRIsure (manufactured by Bioline). The total RNA was reverse transcribed using Primescript (manufactured by Takara Bio) to give a cDNA. Subsequently, quantitative RT-PCR was performed using Light Cycler 480 (manufactured by Roche). The quantitative RT-PCR test was performed using the following primer sequences.
Human MEX3B primer forward: 5'-ACCCAGTTCTGAGCATGTCG-3' (SEQ ID NO: 27)
Human MEX3B primer reverse: 5'-CGAACTGGGGTCTTGATGTAA-3' (SEQ ID NO: 28)
Human GAPDH primer forward: 5'-GCACCGTCAAGGCTGAGAAC-3' (SEQ ID NO: 29)
Human GAPDH primer reverse: 5'-TGGTGAAGACGCCAGTGGA-3' (SEQ ID NO: 30)
GAPDH was an internal standard.

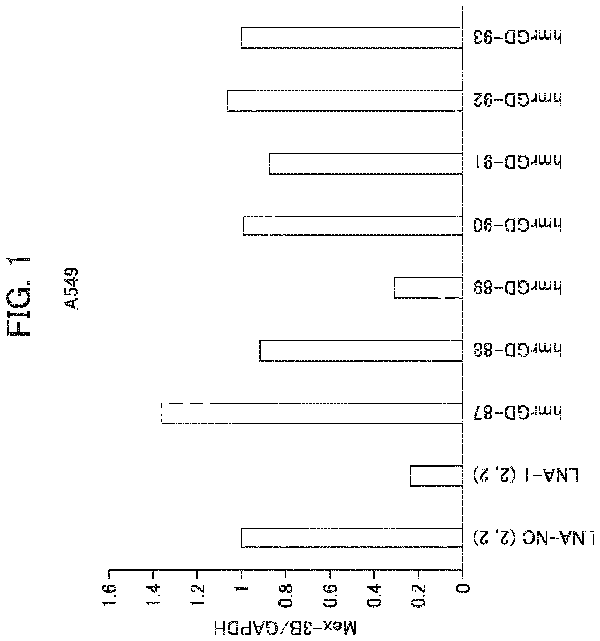

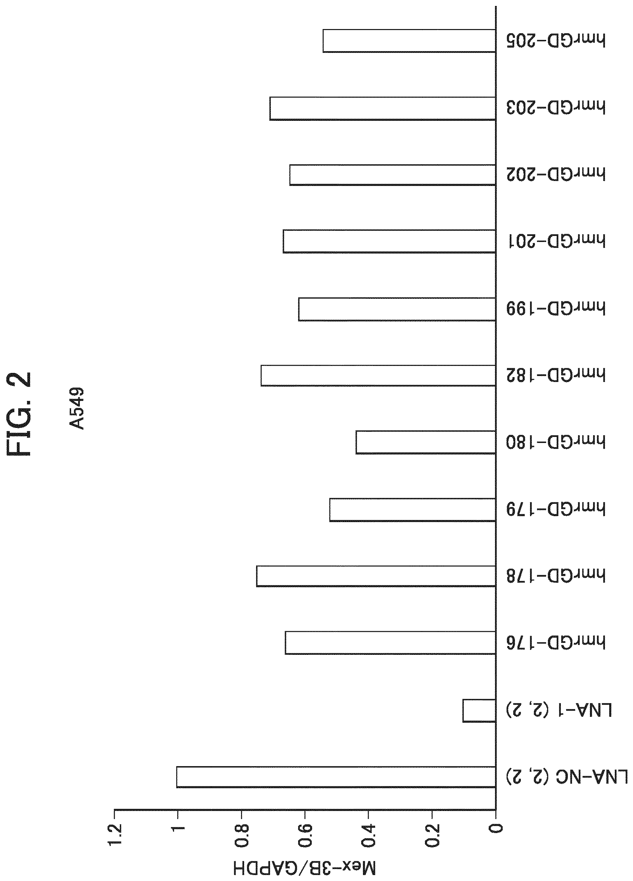

The results of the quantitative RT-PCR shown in FIGS. 1 and 2 indicate that the gapmer-type nucleic acid hmrGD-89 (SEQ ID NO: 2) reduced the expression level to about 1/3 of that for the negative control gapmer-type nucleic acid LNA-NC(2,2) (SEQ ID NO: 10) and showed a human MEX3B mRNA expression-inhibiting effect at a similar level to that for the positive control gapmer-type nucleic acid LNA-1(2,2). The ability to inhibit the expression was remarkably superior to that of other gapmer-type nucleic acids targeting 3'UTR. Although not shown, no adverse effects, such as cytotoxicity, were observed in any case where the cells were transfected with the gapmer-type nucleic acid hmrGD-87, hmrGD-88, hmrGD-89, hmrGD-90, hmrGD-91, hmrGD-92, hmrGD-93, hmrGD-176, hmrGD-178, hmrGD-179, hmrGD-180, hmrGD-182, hmrGD-199, hmrGD-201, hmrGD-202, hmrGD-203, or hmrGD-205, as in the case of the negative control. In the case of the positive control gapmer-type nucleic acid LNA-1(2,2), cytotoxicity was slightly observed.

### Example 2

Since hmrGD-89 (SEQ ID NO: 2) had a significant human MEX3B mRNA expression-inhibiting effect as shown above, gapmer-type nucleic acids shifted by one or two nucleotides from the hmrGD-89 target sequence were prepared and then subjected to the test as in Example 1 in order to search for further gapmer-type nucleic acids having the mRNA expression-inhibiting effect. As summarized in Table 2 below, the gapmer-type nucleic acid hmrGD-89-2 (SEQ ID NO: 1) is such that its target sequence is shifted by two nucleotides to the 5' end from the hmrGD-89 target sequence.

**[Table 2]**

| Gapmer-type nucleic acid | SEQ ID NO | Gapmer sequence | Oligonucleotid e length | | Target sequence | Target region (Human pre-mRNA) | Target region |
|---|---|---|---|---|---|---|---|
| hmrGD-89-2 | 1 | GCGGATACTACAGCTT | 16 | | AAGCTGTAGTATCCGC | 3137-3152 | 3'UTR |
| hmrGD-89 | 2 | GAGCGGATACTACAGC | 16 | - | GCTGTAGTATCCGCTC | 3139-3154 | 3'UTR |

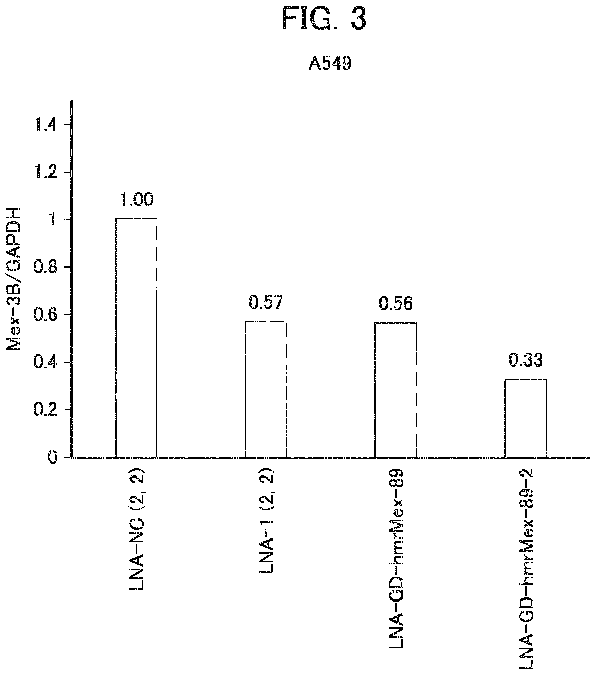

The results of the quantitative RT-PCR shown in FIG. 3 indicate that the gapmer-type nucleic acid hmrGD-89-2 (SEQ ID NO: 1) showed a human MEX3B mRNA expression-inhibiting effect higher than that of the gapmer-type nucleic acid hmrGD-89 (SEQ ID NO: 2), which was at a similar level to that for the positive control gapmer-type nucleic acid LNA-1(2,2). No adverse effects, such as cytotoxicity, were observed in the cells transfected with the gapmer-type nucleic acid hmrGD-89-2 as in the negative control.

### Example 3: Mouse MEX3B mRNA Expression Inhibition assay

The gapmer-type nucleic acids hmrGD-89-2 and hmrGD-89 were subjected to a mouse MEX3B mRNA expression inhibition assay.

### Transfection with Gapmer-Type Nucleic Acid

Transfection with the gapmer-type nucleic acid hmrGD-89-2 or hmrGD-89 was performed as in Example 2 except that the cells to be transfected were altered to MLE-15 mouse pulmonary epithelial cells.

### Quantitative RT-PCR Test

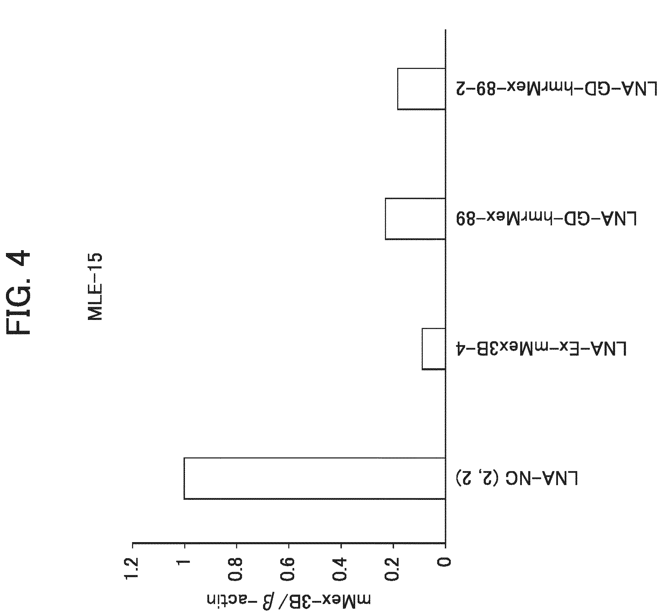

A quantitative RT-PCR test was performed as in Example 2 except that the primers shown below were used instead in the quantitative RT-PCR test. The results are shown in FIG. 4.
Mouse MEX3B primer forward: 5'-CGTCGTCCTCTGTGGTCTTTCCCGGGGGTG-3' (SEQ ID NO: 31)
Mouse MEX3B primer reverse: 5'-TCAGGAAAAAATGCGGATGGCCTGAGTGAC-3' (SEQ ID NO: 32)
Mouse β-actin primer forward: 5'-GGATGCAGAAGGAGATTACTGC-3' (SEQ ID NO: 33)
Mouse β-actin primer reverse: 5'-CCACCGATCCACACAGAGCA-3' (SEQ ID NO: 34)

### β-Actin is an internal standard.

The results of the quantitative RT-PCR shown in FIG. 4 indicate that all of the gapmer-type nucleic acids hmrGD-89-2 and hmrGD-89 had a mouse MEX3B mRNA expression-inhibiting effect at a similar level to that for the positive control gapmer-type nucleic acid LNA-Ex-mMEX3B-4, which is complementary to a given target sequence on the mouse pre-mRNA and is known to exhibit a certain level of MEX3B gene expression-inhibiting effect.

### Example 4: Human MEX3B mRNA Expression Inhibition Assay Based on Free Uptake

A human MEX3B mRNA expression inhibition assay was performed based on free uptake instead of the transfection method using Lipofectamine. The test was performed using not only the LNA gapmer-type nucleic acids hmrGD-89-2 and hmrGD-89, as used in Example 2, but also the ENA gapmer-type nucleic acids hmrGD-89-2 and hmrGD-89, which were prepared separately from the LNA type.

### Transfection through Free Uptake

Each nucleic acid was introduced into cells as in Example 2 except that the Lipofectamine RNAiMAX (manufactured by Invitrogen) was not used and that the nucleic acid was introduced at a final concentration of 1 µM into the cells.

### Quantitative RT-PCR Test

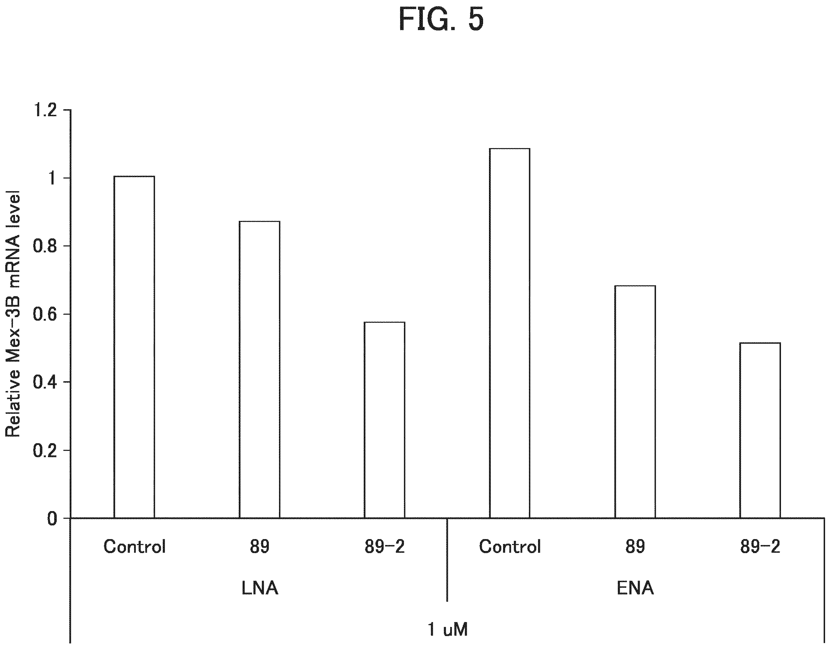

Quantitative RT-PCR was performed as in Example 2 except that the cells were observed for their condition under a microscope 72 hours after the transfection and then the total RNA was collected from the cells. The results are shown in FIG. 5. The "control" shown in FIG. 5 represents a negative control.

The results of the quantitative RT-PCR shown in FIG. 5 indicate that both of the LNA and ENA gapmer-types of hmrGD-89-2 and hmrGD-89 are highly effective in inhibiting human MEX3B mRNA expression even in the case of free uptake and can be expected to inhibit MEX3B gene expression even when they are administered in vivo. In particular, the LNA and ENA types of hmrGD-89-2 were highly effective.

### Example 5: Human and Mouse MEX3B Protein Expression Inhibition Assay

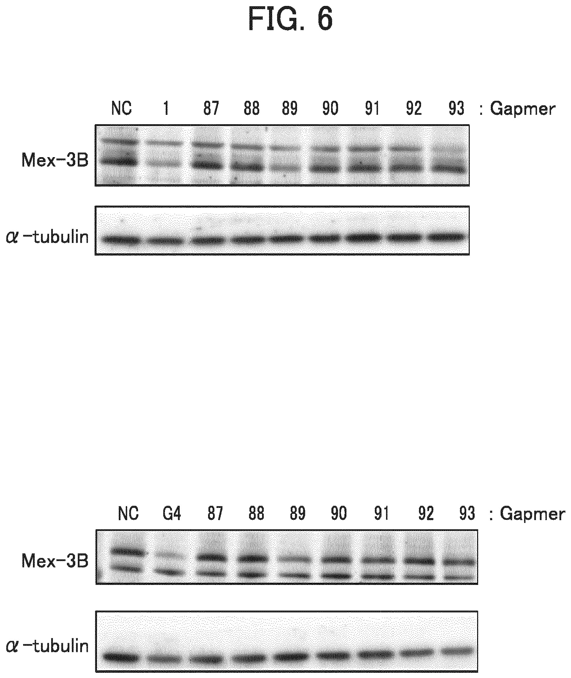

As in Example 1, an MEX3B gene expression inhibition assay was performed comprehensively on the full length of unspliced human MEX3B pre-mRNA. In the assay, not only A549 human alveolar basal epithelial adenocarcinoma cells but also MLE-15 mouse pulmonary epithelial cells were transfected with the nucleic acid, then observed for their condition under a microscope 48 hours after the transfection, and then harvested. Subsequently, the total protein was collected from the cells using a radio-immunoprecipitation assay (RIPA) buffer (manufactured by FUJIFILM Wako Chemicals) and then subjected to human and mouse MEX3B protein analysis using Western blotting. FIG. 6 shows the results of the test using the gapmer-type nucleic acids hmrGD-87 (SEQ ID NO: 11), hmrGD-88 (SEQ ID NO: 12), hmrGD-89 (SEQ ID NO: 2), hmrGD-90 (SEQ ID NO: 13), hmrGD-91 (SEQ ID NO: 14), hmrGD-92 (SEQ ID NO: 15), and hmrGD-93 (SEQ ID NO: 16). In the drawing, part a) shows the results obtained from A549 human alveolar basal epithelial adenocarcinoma cells, and part b) shows the results obtained from MLE-15 mouse pulmonary epithelial cells. α-Tubulin is an internal standard (hereinafter the same applies). Lane NC shows the results obtained when the negative control gapmer-type nucleic acid LNA-NC(2,2) (SEQ ID NO: 10) was used, and both of lanes 1 and G4 show the results obtained when the positive control gapmer-type nucleic acid was used.

The Western blotting results shown in parts a) and b) of FIG. 6 indicate that, similar to the positive control gapmer-type nucleic acid, the gapmer-type nucleic acid hmrGD-89 reduced the signals of both of the human and mouse MEX3B protein bands and inhibited the MEX3B protein expression. This effect was significantly higher than that of other gapmer-type nucleic acids targeting 3'UTR.

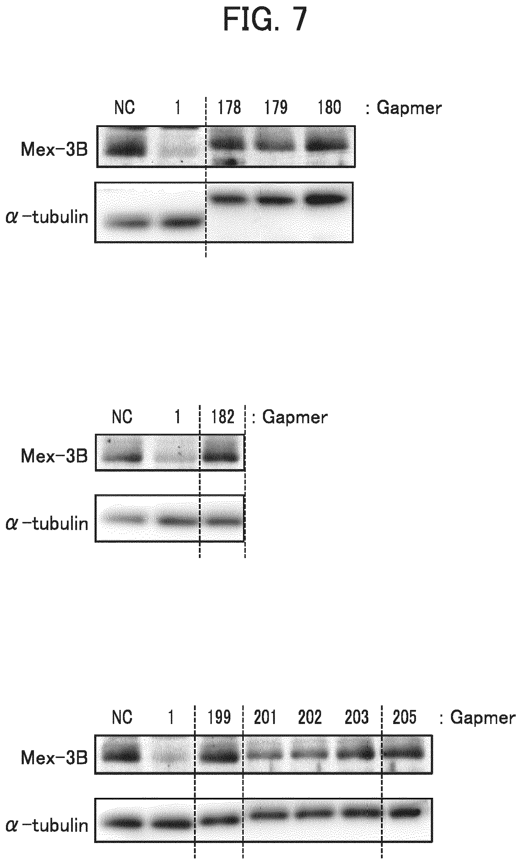

Among the gapmer-type nucleic acids shown in Patent Document 2, hmrGD-178, hmrGD-179, hmrGD-180, hmrGD-182, hmrGD-199, hmrGD-201, hmrGD-202, hmrGD-203, and hmrGD-205 were also used to transfect A549 human alveolar basal epithelial adenocarcinoma cells. Subsequently, human MEX3B protein expression inhibition was examined using Western blotting. The results are shown in FIG. 7. The Western blotting results shown in FIG. 7 indicate that the MEX3B protein band was slightly faint when hmrGD-178, hmrGD-179, hmrGD-180, hmrGD-182, hmrGD-199, hmrGD-201, hmrGD-202, hmrGD-203, or hmrGD-205, in particular, hmrGD-201 or hmrGD-202 was used than when the negative control NC was used.

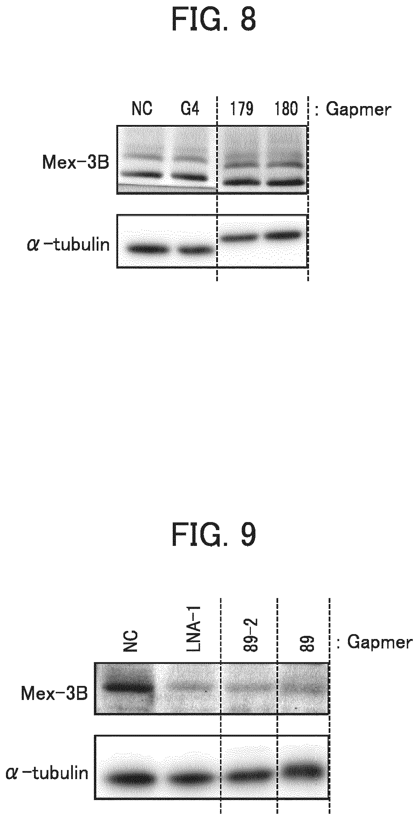

Among the gapmer-type nucleic acids shown in Patent Document 2, hmrGD-179 and hmrGD-180 were used to transfect MLE-15 mouse pulmonary epithelial cells. Subsequently, mouse MEX3B protein expression inhibition was examined using Western blotting. The results are shown in FIG. 8. The Western blotting results shown in FIG. 8 indicate that the MEX3B protein band was slightly faint when hmrGD-179 or hmrGD-180 was used than when the negative control NC was used.

### Example 6: Human and Mouse MEX3B Protein Expression Inhibition Assay

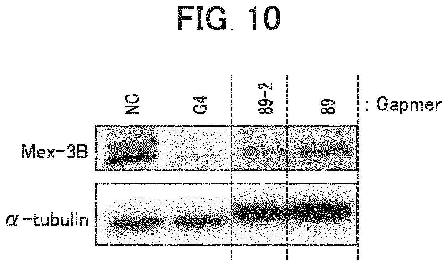

Since hmrGD-89 had a significant MEX3B protein expression-inhibiting effect as shown above, hmrGD-89-2 was also subjected to human and mouse MEX3B protein expression inhibition assay based on Western blotting as in Example 5. FIGS. 9 and 10 show the results of the test on human MEX3B and mouse MEX3B, respectively.

The results shown in FIGS. 9 and 10 indicate that the gapmer-type nucleic acids hmrGD-89-2 and hmrGD-89 reduced the signals of both of the human and mouse MEX3B protein bands as the positive control gapmer-type nucleic acid did, which suggests significant inhibition of the MEX3B protein expression.

### [Sequence Listing]

ATF-491PCT ST25.txt

## Claims

1. A nucleic acid selected from the following oligonucleotides:
(1) an oligonucleotide consisting of the nucleotide sequence set forth in SEQ ID NO: 1 or 2 in the Sequence Listing;
(2) an antisense oligonucleotide that consists of a nucleotide sequence comprising a deletion, substitution, and/or addition of one or two nucleotides with respect to the nucleotide sequence set forth in SEQ ID NO: 1 or 2 in the Sequence Listing and inhibits MEX3B gene expression; and
(3) an antisense oligonucleotide that comprises the nucleotide sequence set forth in SEQ ID NO: 3 in the Sequence Listing and inhibits MEX3B gene expression.

2. The nucleic acid according to claim 1, wherein the antisense oligonucleotide (2) consists of a nucleotide sequence having a deletion of one or two nucleotides with respect to the nucleotide sequence set forth in SEQ ID NO: 1 or 2 in the Sequence Listing.

3. The nucleic acid according to claim 1, wherein the antisense oligonucleotide (3) has a sequence of 15 or less nucleotides.

4. A nucleic acid for use as an MEX3B gene expression inhibitor, the nucleic acid comprising the nucleic acid according to any one of claims 1 to 3.

5. A method for inhibiting MEX3B gene expression, the method comprising contacting, in vitro, the inhibitor according to claim 4 with a subject, excluding a human individual, wherein the subject is a microorganism, a protozoan, a biological tissue section, a human cell, or an animal cell.

6. A nucleic acid for use as a prophylactic or therapeutic agent for a disease caused by MEX3B gene expression, the nucleic acid comprising the nucleic acid according to any one of claims 1 to 3.

## Patentansprüche

1. Nukleinsäure, ausgewählt aus den folgenden Oligonukleotiden:
(1) einem Oligonukleotid, das aus der Nukleotidsequenz gemäß SEQ ID NO: 1 oder 2 im Sequenzprotokoll besteht;
(2) einem Antisense-Oligonukleotid, das aus einer eine Deletion, Substitution und/oder Addition von einem oder zwei Nukleotiden in Bezug auf die Nukleotidsequenz gemäß SEQ ID NO: 1 oder 2 im Sequenzprotokoll umfassenden Nukleotidsequenz besteht und die Expression des MEX3B-Gens hemmt; und
(3) einem Antisense-Oligonukleotid, das die Nukleotidsequenz gemäß SEQ ID NO: 3 im Sequenzprotokoll umfasst und die Expression des MEX3B-Gens hemmt.

2. Nukleinsäure nach Anspruch 1, wobei das Antisense-Oligonukleotid (2) aus einer Nukleotidsequenz mit einer Deletion von ein oder zwei Nukleotiden in Bezug auf die Nukleotidsequenz gemäß SEQ ID NO: 1 oder 2 im Sequenzprotokoll besteht.

3. Nukleinsäure nach Anspruch 1, wobei das Antisense-Oligonukleotid (3) eine Sequenz von 15 oder weniger Nukleotiden aufweist.

4. Nukleinsäure zur Verwendung als Hemmer der Expression des MEX3B-Gens, wobei die Nukleinsäure die Nukleinsäure nach einem der Ansprüche 1 bis 3 umfasst.

5. Verfahren zur Hemmung der Expression des MEX3B-Gens, wobei das Verfahren In-Kontakt-Bringen des Hemmers nach Anspruch 4 in vitro mit einem Subjekt, mit Ausnahme eines Menschen, umfasst, wobei es sich bei dem Subjekt um einen Mikroorganismus, ein Protozoon, einen biologischen Gewebeschnitt, eine menschliche Zelle oder eine tierische Zelle handelt.

6. Nukleinsäure zur Verwendung als Prophylaktikum oder Therapeutikum gegen eine durch die Expression des MEX3B-Gens verursachte Krankheit, wobei die Nukleinsäure die Nukleinsäure nach einem der Ansprüche 1 bis 3 umfasst.

## Revendications

1. Acide nucléique choisi parmi les oligonucléotides suivants :
(1) un oligonucléotide constitué de la séquence nucléotidique présentée dans SEQ ID NO: 1 ou 2 dans la liste des séquences ;
(2) un oligonucléotide antisens qui est constitué d'une séquence nucléotidique comprenant une délétion, une substitution et/ou une addition d'un ou deux nucléotides par rapport à la séquence nucléotidique présentée dans SEQ ID NO: 1 ou 2 dans la liste des séquences et inhibe l'expression du gène MEX3B ; et
(3) un oligonucléotide antisens qui comprend la séquence nucléotidique présentée dans SEQ ID NO: 3 dans la liste des séquences et inhibe l'expression du gène MEX3B.

2. Acide nucléique selon la revendication 1, dans lequel l'oligonucléotide antisens (2) est constitué d'une séquence nucléotidique ayant une délétion d'un ou deux nucléotides par rapport à la séquence nucléotidique présentée dans SEQ ID NO: 1 ou 2 dans la liste des séquences.

3. Acide nucléique selon la revendication 1, dans lequel l'oligonucléotide antisens (3) a une séquence de 15 nucléotides ou moins.

4. Acide nucléique destiné à être utilisé comme inhibiteur d'expression du gène MEX3B, l'acide nucléique comprenant l'acide nucléique selon l'une quelconque des revendications 1 à 3.

5. Procédé pour inhiber l'expression du gène MEX3B, le procédé comprenant la mise en contact, *in vitro,* de l'inhibiteur selon la revendication 4 avec un sujet, à l'exclusion d'un individu humain, dans lequel le sujet est un micro-organisme, un protozoaire, une coupe de tissu biologique, une cellule humaine ou une cellule animale.

6. Acide nucléique destiné à être utilisé comme agent prophylactique ou thérapeutique pour une maladie causée par l'expression du gène MEX3B, l'acide nucléique comprenant l'acide nucléique selon l'une quelconque des revendications 1 à 3.
